(12) **EUROPEAN PATENT SPECIFICATION**

(45) Date of publication and mention
of the grant of the patent:
**10.04.2019 Bulletin 2019/15**

(51) Int Cl.:
***A61M 16/06*** *(2006.01)*        ***A61M 16/08*** *(2006.01)*
***A61B 5/08*** *(2006.01)*

(21) Application number: **16183417.1**

(22) Date of filing: **09.08.2016**

(54) **NASAL CANNULA**

NASENKANÜLE

CANULE NASALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.05.2016 US 201662336916 P**

(43) Date of publication of application:
**22.11.2017 Bulletin 2017/47**

(73) Proprietor: **L'AIR LIQUIDE, SOCIETE ANONYME
POUR
L'ETUDE ET L'EXPLOITATION DES PROCEDES
GEORGES CLAUDE
75007 Paris (FR)**

(72) Inventor: **BOULANGER, Thierry
Swarthmore, PA 19081 (US)**

(74) Representative: **Pittis, Olivier
L'Air Liquide, S.A.
Direction de la Propriété Intellectuelle
75, Quai d'Orsay
75321 Paris Cedex 07 (FR)**

(56) References cited:
**EP-A1- 2 075 023          EP-A1- 2 233 167
EP-A1- 2 720 005          EP-A1- 2 992 920
WO-A1-97/18003           WO-A1-2015/156690
WO-A2-2012/037469      US-A- 4 723 543
US-A- 4 989 599            US-A- 5 099 836**

**Description**

The invention concerns a gas delivery installation or assembly comprising a nasal sub-system or nasal gas-delivery system comprising two prongs that are inserted into the patient's nostrils for delivering a respiratory gas to a patient in need thereof, and a sensing module, and preferably a processing unit and a high flow generator (HFG) for delivering a respiratory gas to a patient.

Nasal High Flow Therapy or NHFT is a widespread therapy used in many hospitals, especially in acute care services, for treating respiratory distresses. It does not require any mechanical ventilation and can prevent patients from being intubated.

Basically, NHFT consists in providing a humidified, high flow of a respiratory gas to a patient in need thereof. Gaseous flows typically range from 10 to 60 L/min in adults and 1 to 10 L/min in infants.

The gas is delivered by a nasal cannula system that typically comprises two prongs that are inserted into the patient's nostrils, while not totally occluding them. The prongs deliver the gaseous flow that the patient inhales and the space that exists between the outer portion of the prongs and the inner wall of the nostrils helps the expired gaseous flow to be vented upon exhalation. An exemplary NHFT device is known from EP2075023A1.

NHFT provides many advantages compared to continuous oxygen treatments or even to non-invasive ventilation, e.g.:

- the gaseous flow can be enriched with Oxygen ($O_2$) up to 100%, i.e. the respiratory gas can be composed of air, $O_2$-enriched air (>22 vol% of $O_2$) or pure $O_2$;
- the set flow can be greater than the inspiratory peak flow of the patient so as to limit his/her work while breathing, thereby assisting preventing fatigue of respiratory muscles;
- the flow helps flushing out $CO_2$ from the dead space of the upper airways, thereby enhancing the ventilation; and
- the flow and the space between the prongs and the nostrils create a positive pressure, which exhibits a beneficial action on ventilation;

Furthermore, this therapy has many advantages such as:

- only a gas flow and a level of oxygen have to be set by the physician or other medical service provider;
- the nasal cannula is easy to be put in place; and
- the patient can speak and even eat, while receiving the therapy.

However, so far, physicians or similar do not have easily access to parameters that might help them for setting the right gas flow and gas concentration(s) for thereby obtaining the best therapy for a given patient, as they usually rely only on oxygen saturation, i.e. $SpO_2$.

Indeed, in some cases, a physician may decide to increase the $O_2$ concentration for reaching an adequate $O_2$-saturation in the blood of the patient, whereas increasing the overall flow of gas would have been a better solution if the patient inspiratory flow was greater than the set one.

Furthermore, despite NHFT, it has been noticed that some patients under NHFT persist in their respiratory distress and have to be intubated eventually. One indicator of a possible future intubation is a persistent dyspnea despite the NHFT therapy or a modification of the minute volume ventilation. However, such an indicator is not easily available to physicians or similar.

Having access to these useful parameters, such as minute ventilation and the respiratory rate, would be valuable for physicians or similar as they could be used for better controlling NHFT in better setting or adjusting the parameters of the treatment or anticipating failure of the therapy and initiating in a timely manner another therapy, such as Non Invasive Ventilation.

It is one purpose of the present invention to improve NHFT in providing additional parameters to physicians or similar, for helping them to better assess the patient condition and better adjust the "settings" of the treatment thereby improving the efficiency of the treatment of the patient so as to avoid or limit the intubation rate, and/or to reduce the length of stay of the patient in hospital, in case of intubation.

Another goal of the present invention is to provide a nasal high flow therapy (NHFT) assembly for providing an efficient NHFT to patients.

One solution according to the present invention concerns a nasal high flow therapy (NHFT) assembly suitable for providing a NHFT to patients, comprising:

A) a nasal gas-delivery system to be connected to a nose of a patient for delivering a respiratory gas to the patient, comprising:

- a hollow body with an inner chamber comprising an inlet for receiving a respiratory gas, and
- a pair of nasal prongs in fluid communication with the inner chamber of the hollow body, each nasal prong comprising a first inner passage fluidly connecting the internal chamber of the hollow body with a nostril of the patient, and
- the hollow body comprises a pair of second passages for fluidly connecting a nostril with a vent conduit arranged in the hollow body, said vent conduit comprising one venting port, and

B) a sensing module comprising a hollow body comprising an internal chamber with an inlet orifice, an outlet orifice, a first port, a second port and an inner wall arranged between the first and second ports and radially projecting into the internal chamber thereby forming a restriction of passage for the gas in said internal chamber, said first and second ports each comprising an exit orifice, the inlet orifice of the sensing module being fluidly connected to the venting port of the vent conduit of the nasal gas-delivery system.

Depending on the embodiment the nasal high flow therapy (NHFT) assembly of the present invention can comprise one or more of the following features:

- a pair of flexible flanges are arranged around the nasal prongs of the nasal gas-delivery system thereby ensuring gas-tightness when the nasal prongs are inserted into the patient's nostrils.
- the nasal prongs have a tronconical shape.
- flexible flanges are made of silicon or any other suitable flexible material.
- the inner wall radially projecting into the internal chamber of the nasal gas-delivery system has a disk-shape with a central orifice.
- the pair of flexible flanges of the nasal gas-delivery system comprises a common flexible wall.
- the nasal gas-delivery system further comprises a fixing system for maintaining the nasal prongs in position into the patient's nostrils.
- the assembly further comprises a processing unit comprising a first pressure sensor and a second pressure sensor, electrically connected to a controller; a controller for processing information provided by the first and second pressure sensors; the first pressure sensor and a second pressure sensor being connected to the internal gas circuit, said internal gas circuit being in fluid communication with the first port of the sensing module.
- the internal gas circuit of the processing unit is in fluid communication with the first port of the sensing module by a first tubing.
- the second pressure sensor of the processing unit is a differential pressure sensor.
- the second pressure sensor of the processing unit is connected to the first and second ports of the sensing module by the first tubing and a second tubing, i.e. hoses, gas conducts or similar.
- the controller is electrically connected to a visual interface for displaying information, such as a display screen.
- the assembly further comprises a high flow generator (HFG) for delivering a high flow of gas, said HFG being in fluid communication with the inlet of the inner chamber of the hollow body of the nasal gas-delivery system.
- the HFG is fluidly connected to the inlet of the inner chamber of the hollow body of the nasal gas-delivery system by a flexible tubing, such as hoses or gas conducts.
- the sensing module is a Pitot-type sensor or any other suitable sensor.

Further, the present invention also concerns a method of analyzing breathing activity of a patient whose nose is connected to a nasal gas-delivery system as described hereabove, i.e. a nasal gas-delivery system according to the present invention, the method comprising the steps of:

a) measuring a baseline respiratory flow Qb flowing through the sensing module,
b) defining an upper value of 1.1*Qb,
c) defining a lower limit of 0.9*Qb,
d) during patient breathing through the nasal gas-delivery system, measuring a breathing associated flow through the sensing module,
e) determining if the breathing associated flow through the sensing module is below the lower limit and/or above the upper limit,
f) when the breathing associated flow through the sensing module is below the lower limit, determining the net volume of flow attributable to gas drawn off by the patient Vi0, wherein a preset threshold for Vi0 is defined as indicative of an excessive patient breathing effort,
g) when the breathing associated flow through the sensing module is above the upper limit, determining if the net volume of exhaled flow Ve attributable to excess flow from the nasal gas-delivery system exceeds a predetermined amount or a predetermined amount for a predetermined number of times within a predefined period.

Other features, aspects and advantages of the present invention will become apparent from the following detailed description when taken in conjunction with the accompanying drawings which illustrates, by way of examples, the present invention, among which:

- Fig. 1 is a schematic representation of a gas delivery assembly comprising a nasal interface according to the prior art,
- Fig. 2 is a schematic representation of the nasal interface of Fig. 1,
- Fig. 3 represents a gas delivery assembly comprising a nasal interface and a sensing module,
- Fig. 4 represents an embodiment of the nasal interface and the sensing module according to the present invention,
- Fig. 5 represents a cross section of the sensing module according to the present invention,
- Fig. 6 represents a cross section of the nasal interface and the sensing module according to the present invention,
- Fig. 7 represents an embodiment of a processing unit of the gas delivery assembly of Fig. 3,
- Fig. 8 represents an acquisition of the breathing pattern of a patient with the sensing module and processing unit,
- Fig. 9 represents another acquisition of the breathing pattern of a patient with the sensing module and processing unit, and

- Fig. 10 is an algorithm running in the processing unit,
- Fig. 11 represents another embodiment of the nasal interface and the sensing module according to the present invention,
- Fig. 12 represents a cross section of Fig.11,
- Fig. 13 represents another acquisition of the breathing pattern of the patient with the sensing module and processing unit,
- Fig. 14 is another algorithm running in the processing unit,
- Fig. 15 is another embodiment of the gas delivery assembly comprising a nasal interface and a sensing module, and
- Fig. 16 is another embodiment of the processing unit of the gas delivery assembly of Fig.15.

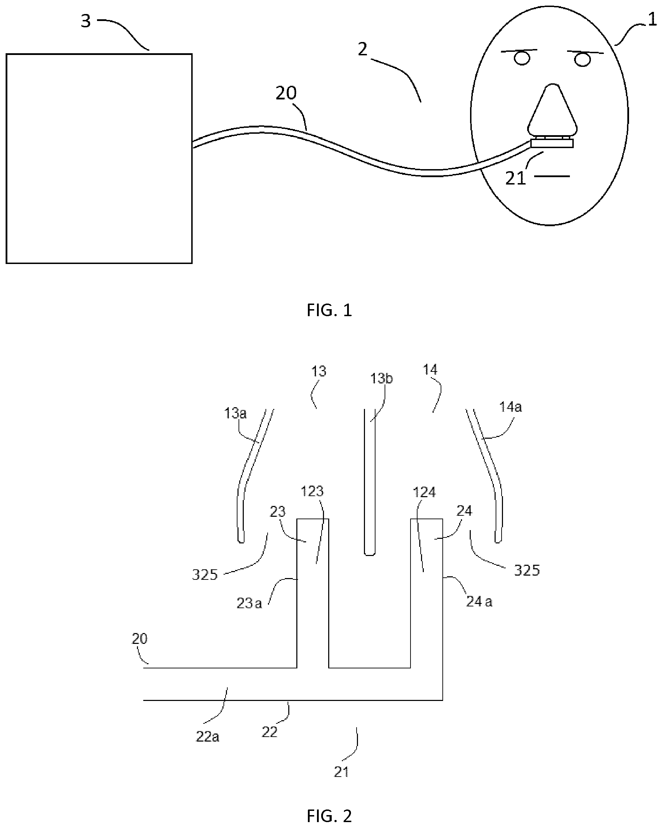

Fig 1 shows a gas delivery equipment comprising a cannula assembly 2 according to the prior art that fluidly connects a patient 1 to a high flow generator 3 or "HFG", such as the device called Optiflow™ commercialized by Fisher & Paykel.

The cannula assembly 2 comprises an elongated flexible tubing 20, such as a flexible hose, comprising, at one end, a nasal sub-system or nasal gas-delivery system 21 fluidly connected to the nose of patient 1, thereby delivering a respiratory gas to the patient 1. The elongated flexible tubing 20 is fluidly connected, at its other end, to the HFG 3.

As detailed in Fig. 2, the nasal sub-system 21 comprises a hollow body 22 with an inner chamber 22a, acting as a manifold for receiving the respiratory gas conveyed and fed by the flexible tubing 20, and nasal prongs 23, 24, i.e. little nozzles, in fluid communication with the lumen of the inner chamber 22a of the hollow body 22, that are inserted, in use, into the nostrils of the patient 1 for delivering a respiratory gas to the patient 1 in need thereof. Each nasal prong 23, 24 comprises a unique inner channel or passage 123, 124 for conveying the gas from the inner chamber 22a of the hollow body 22 to the nostrils 13, 14 of the patient 1.

The hollow body 22 can have various shapes, for example be of circular or rectangular cross section. The gas fed by the tubing 20 travels successively in the chamber 22a of the hollow body 22 and then in each nasal prongs 23, 24, and eventually is distributed into the nostrils 13, 14 of the patient.

With such a prior art equipment, a gas tight connection/insertion of the prongs 23, 24 into the nostrils 13, 14 is not ensured as the outer peripheral walls 23a, 24a of the nasal prongs 23, 24, respectively, are not operated in a manner that provides a sealed contact with the inner walls 13q, 14a of the nostrils 13, 14, respectively. This means that a spacing 325 always exists between the inner walls 13q, 14a of the nostrils 13, 14, and the outer peripheral walls 23a, 24a of the nasal prongs 23, 24, respectively, leading to gas loses.

The gaseous flow delivered by the high flow generator 3 enters into the inner chamber 22a of the hollow body 22 and is then directed to the prongs 23, 24 so that upon inhalation, only a part of the gaseous flow is inspired by the patient 1, as the rest is unfortunately vented to the atmosphere, while escaping through the spacing 325 that inevitably exists.

Upon expiration, both gaseous flows coming, on the one hand, from the high flow generator 3 and, on the other hand, exhaled by the patient do circulate in said spacing 325, thereby creating a positive expiratory pressure before being vented to the atmosphere.

Such an architecture is not ideal because such gas loss cannot be quantified and therefore prevent the access to precious respiratory data such as volumes.

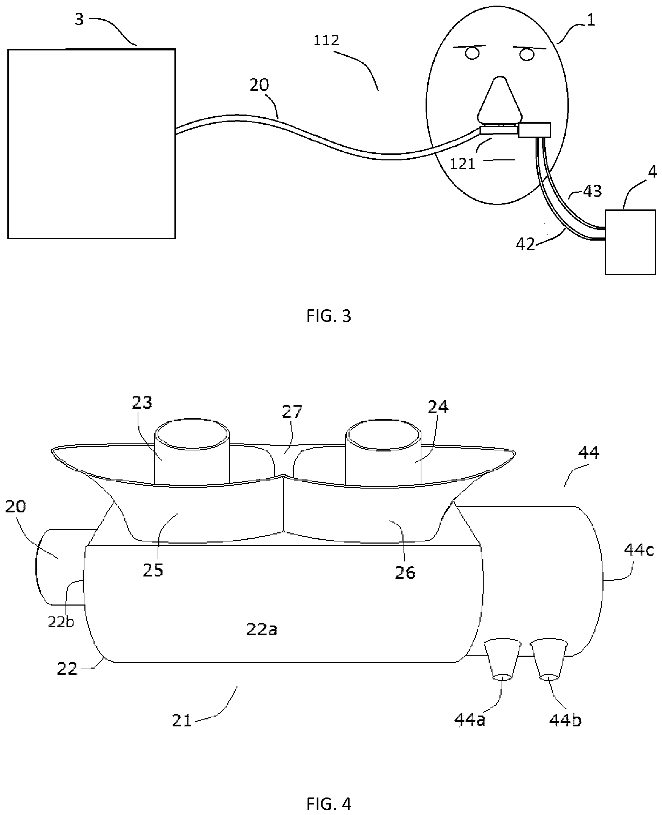

Figure 3 represents a first embodiment of a gas delivery equipment comprising a first embodiment of a cannula assembly 112 according to the present invention for fluidly connecting a patient 1 to a HFG 3. As in Figures 1 and 2, the cannula assembly 112 comprises an elongated flexible tubing 20 connected, on the one hand, to the high flow generator 3, and, on the other hand, to a nasal sub-system or nasal gas-delivery system 121 inserted into the nose of patient 1, thereby delivering respiratory gas to the patient 1.

According to the present invention, the nasal gas-delivery system 121 further comprises measurement elements or means for measuring and transmitting physiological signals, such as gas pressure and/or flowrate, to a processing unit 4. These additional measurement elements or means comprise three measurement lines, namely flexible tubings 42, 43 that are fluidly connected to the nasal gas-delivery system 121 as shown in Figure 3.

Figure 4, shows an embodiment of a nasal gas-delivery system 21 according to the present invention. It comprises a hollow body 22 having any suitable shape, for example a circular or rectangular section, and providing an inlet 22b receiving a respiratory gas fed by the flexible tubing 20, whereas the outlet orifice 44c, part of a sensing module 44, recovers the expiratory gas of the patient.

The nasal gas-delivery system 21 further comprises two nasal prongs 23, 24 which deliver the gas to the patient, that comprises two deformable flanges 25, 26 separated by a common wall 27 for performing a gas tight seal with the patient's nose and for thus better recovering the gas expired by the patient.

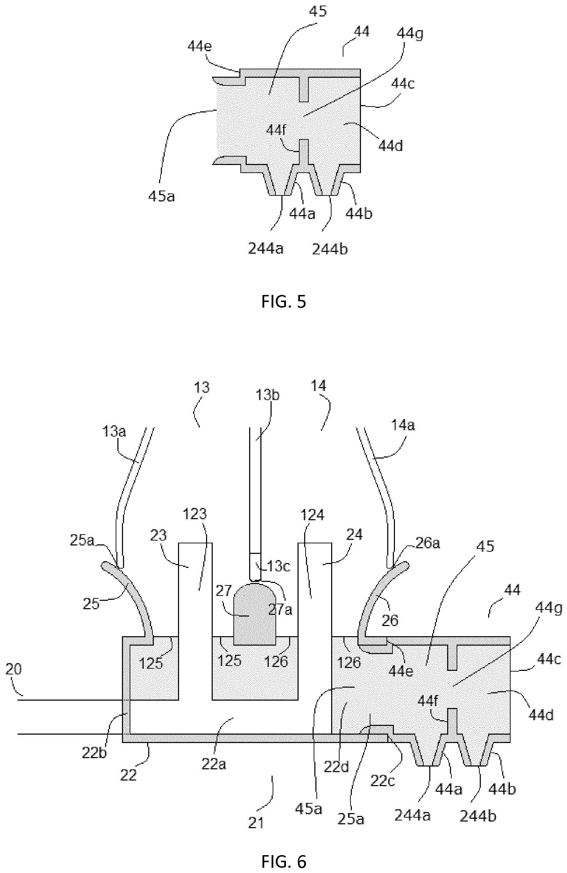

Further, the sensing module 44 comprises two ports 44a, 44b which will give access to the flow measurement, as explained in connection with Figure 5.

Fig. 5 is a cross sectional view of an embodiment of a sensing module 44. The sensing module 44 comprises, in the present embodiment, a circular, hollow body 45 comprising an internal chamber 44d and an inner wall 44f arranged in that internal chamber 44d. The inner wall 44f restricts locally the diameter of the chamber 44d, i.e. the passage for the gas, thereby forming a restriction of passage 44g and thus creating a pressure drop when a gaseous flow circulates in chamber 44d. First and second ports 44a, 44b are provided to fluidly connect the internal chamber 44d upstream and downstream of the restriction formed by inner wall 44f. Both first and second ports 44a, 44b, respectively, comprise an exit orifice 244a, 244b, respectively, through which the gas can exit the internal chamber 44d. First port 44a is itself fluidly connected to a first flexible tubing 42 for feeding gas to the first flexible tubing 42, whereas second port 44b is itself fluidly connected to a second flexible tubing 43 for feeding gas to the second flexible tubing 43.

Figures 4 and 6 show the sensing module 44 fluidly connected to the nasal gas-delivery system 21 of Figure 3.

Fig. 6 is a cross sectional view showing the inner design of the nasal gas-delivery system 21 of Figure 3 and the sensing module 44 fluidly connected to said nasal gas-delivery system 21 and locked and maintained therein thanks to the contact between edges 44e, 22c.

The nasal prongs 23, 24 are inserted into the patient's nostrils 13, 14. The inlet 22b of the main hollow body of the nasal gas-delivery system 21, that receives the gas from the flexible tubing 20, is in fluid communication with a first inner chamber 22a that is itself in fluid communication with the inner passages 123, 124 of the prongs 23, 24 so that gas can circulate from the tubing 20 to the prongs 23, 24, via the first inner chamber 22a, and can thereby be delivered to the nostrils 13, 14. Similarly, conduits 125, 126 are in fluid communication with a second inner chamber 22d arranged in the main body of the nasal gas-delivery system 21, that comprises a gas outlet orifice 44c. From the gas outlet orifice 44c, the gases can be easily collected.

Further, the nasal gas-delivery system 21 comprises two flanges 25, 26 ensuring gas tightness with the outer regions of the nose of the patient. Indeed, nostrils 13, 14 comprise outer walls 13a, 14a, also called "ala" of the nose, and the nasal septum 13b ends by the columella 13c as illustrated in Fig. 6. Flexible flange 25 of the nasal gas-delivery system 21 of the invention, which is deformable, comes into contact with the ala 13q (in 25a), whereas the flange 26 comes into contact with the ala 14a (in 26a), and whereas flexible wall 27, which also comprises a deformable portion, comes into contact with the columella 13c (in 27a). Those contact areas 25a, 26a, 27a create a gas seal around the nostrils 13, 14, i.e. on the border delimiting the nostril's entrance.

With such a configuration, the gaseous flow, such as air, conveyed by the tubing 20 enters into the internal chamber 22a of the hollow body 22, travels through the first and second conduits 123, 124 of the pair of prongs 23, 24 and is then delivered to the nostrils 13, 14 of the patient.

During the inhalation phases of the patient, a part of the gaseous flow is inspired by the patient, whereas the rest of the gaseous flow is directed to the conduits 125, 126 which are fluidly connected to the vent conduit 22d and subsequently to internal chamber 44d thereby allowing this excess of gas escaping to the atmosphere by venting port(s) 44c.

In contrast, during the exhalation phases of the patient, both gaseous flows coming from the internal chamber 22a of the hollow body 22, on the one hand, and exhaled by the patient, on the other hand, pass through the conduits 125, 126 and are vented to the atmosphere thanks to vent conduits 22d and subsequently to internal chamber 44d, and venting port or gas outlet orifice 44c, thereby creating a positive expiratory pressure or PEP.

The nasal gas-delivery 21 can be made of soft silicon material so as to be light for the patient. Further, it preferably comprises one or several straps, a headgear or similar a fixing system (not shown) for maintaining the nasal gas delivery system 21 in position in the nostrils 13, 14 of the patient.

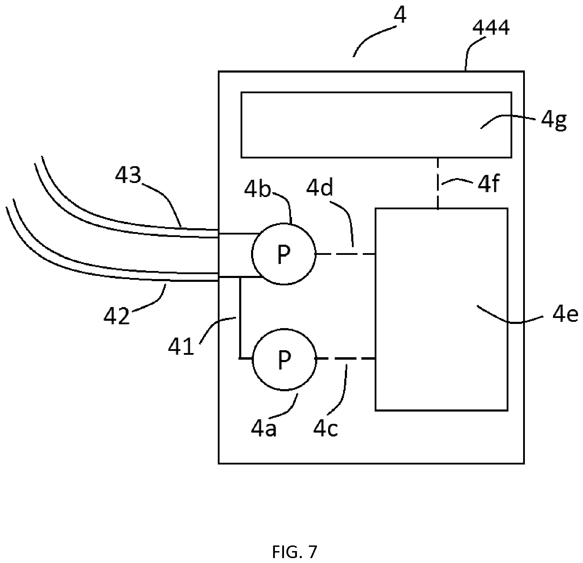

In Fig. 7, the flexible tubing 42, 43 are themselves fluidly connected to processing unit 4, which comprises processing means or devices for processing signal(s), such as one or several microcontrollers.

As illustrated in Fig. 5, the gas travelling into the internal chamber 44d is submitted to a slight pressure drop during its propagation towards the second and third ports 44a, 44b due to the presence of the internal wall 44f, as above explained. Such a structure constitutes a flow sensor so that gas pressure measurements can be made through the second and third ports 44a, 44b and then forwarded to the processing unit 4 by the flexible tubings 42, 43, where they can be processed.

The processing unit 4 comprises a casing 444 that comprises two pressure sensors 4a, 4b, i.e. a first pressure sensor 4a and a second pressure sensor 4b. First sensor 4a is connected to an internal tubing or gas circuit 41 which is fluid communication with tubing 42 and port 44a of sensing module 44. The first pressure sensor 4a measures the pressure that exists close to the patient's nostrils 13, 14. First pressure sensor 4a is electrically connected, e.g. with cable 4c, to a controller 4e which will further transform and process the pressure information or signal delivered by the first sensor 4a. For instance, the average pressure over a given period of time, such as 1 sec, will help detect a partial occlusion of the passage 44g due to the accumulation of secretions.

Similarly, the second pressure sensor 4b is connected to first and second tubing 42, 43 which are respectively connected to the first and second ports 44a, 44b of the sensing module 44.

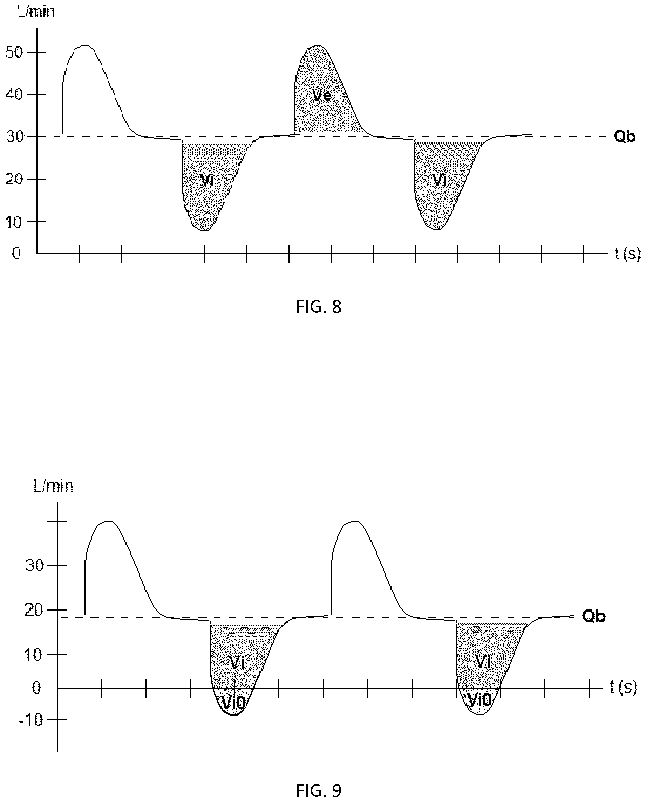

The second pressure sensor 4b is preferably a differential pressure sensor allowing measuring the slight pressure difference, i.e. pressure drop, that exists between tubing 42, 43, said pressure drop resulting from the gas pressure decrease caused by the inner wall 44f of the sensing module 44. The second sensor 4b is electrically connected, e.g. with cable 4d, to controller 4e which will, here again, further transform and process the pressure information or signal delivered by the second sensor 4b. For instance, based on the geometry of the inner chamber 44d and wall 44f, controller 4e is able to determine the absolute value of the flow, relying on a stored lookup table, for translating the differential pressure readings from pressure sensor 4b to actual gas flow values. Fig. 8 illustrates the gaseous flow (in L/min) that is measured by unit 4 over the time (in sec). As one can see, the flow is swinging around a mean value Qb (i.e. an average flow value) representing the flow coming off the HFG 3. Upon inhalation, most of the flow coming from the HFG 3 will be inspired by the patient 1 and the instantaneous flow traveling in the chamber 44d will fall below the average flow value. On the opposite, during expiration, the flow of the patient will sum up with the flow from the HFG 3 and the instantaneous flow traveling in the chamber 44d will rise above the average flow value.

Fig. 9 is a similar illustration of the gaseous flow (in L/min) that is measured by the unit 4 over the time (in sec). The difference here is that there is a period of time during inspiration phases, where the flow becomes negative. This means that the demand of the patient exceeds the flow delivered by the HFG 3 and that flow comes backward that is sucked from ambient air by the patient 1, traveling from inner chamber 44d to exhaust ports 125, 126 of the nasal prongs. This situation indicates that the flow delivered by the HFG 3 is inferior to inspiratory peak flow of the patient 1, increasing his work of breathing. This should be notified to the physician.

Similarly, being able to calculate the mean value of the flow coming from the HFG 3 and the amplitude of the flow swings above and under this limit would give access to the inspired volume Vi that is inhaled and to the expired volume Ve that is exhaled by the patient 1 as well as to the respiratory rate of the patient 1.

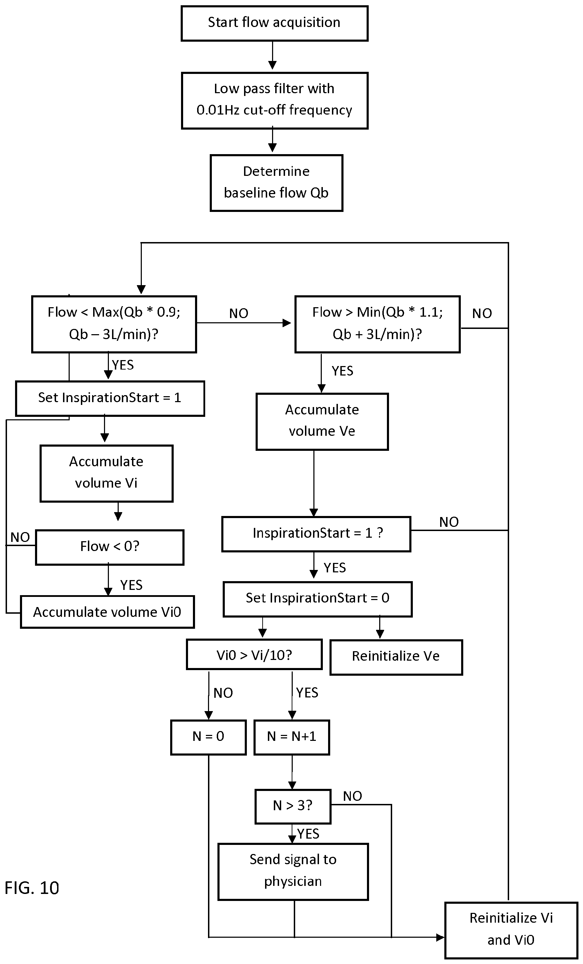

Fig. 10 is the description of one algorithm that could be implemented in unit 4 and operated by the controller 4e to feed the medical team with useful information.

As the flow therapy is started, a low-pass filter with a cut-off frequency within the range of 0.005 to 0.1 Hz, typically 0.01 Hz is applied. This has the benefit of removing the breathing pattern of the patient 1 whose frequency is usually in the range of 0.1 to 1 Hz and gives access to the baseline flow Qb representing the flow delivered by the HFG 3.

Such portion of the algorithm is continuously running in order to take into account any setting or adjustment of the flow that has to be delivered by the High Flow Generator 3.

This value of Qb is then used to define two limits comprising an upper limit and a lower limit, the lower one being slightly lower and the upper one being slightly greater than Qb.

These upper and lower limits will determine whether the patient is inhaling (i.e. lower values) or exhaling (i.e. upper values). The lower value is set as the maximum of 90% of Qb or Qb - 3 L/min, whereas the upper limit is set as the minimum of 110% of Qb or Qb + 3L/min.

For example, if Qb is determined as 40 L/min, the lower limit is 37 L/min and the upper limit is 43 L/min.

At any moment, once Qb has been determined, the controller 4e verifies the conditions. In case the flow goes below the set lower limit, the volume Vi is accumulated over time, when the flow becomes greater than the low-limit the accumulation is terminated and the tidal volume of the patient 1 is calculated. It is important to note that in the same period of time, a second algorithm checks whether the flow is negative to determine the volume Vi0 the patient would have breath on his/her own due to a demand exceeding the flow Qb delivered by the HFG 3.

When the flow is between the lower and upper limits, there is no specific action performed in terms of volume calculation. However, as soon as the patient starts exhaling, the flow quickly exceeds the upper limit and the volume Ve is accumulated over time.

In the meantime a specific algorithm will check the values Vi0 and Vi calculated during the previous inspiration.

If more than 10% of the volume Vi0 was drawn by the patient, this exposes him/her to subsequent respiratory fatigue. In case of 3 occurrences in a row, i.e. 3 consecutives inspiration, a signal will be sent to the medical team, asking them to check the flow delivered by the HFG 3 and increase its value, if necessary.

In order to send such signal and display all the parameters (pressure, respiratory rate, inspiratory volumes and minute volume ...), controller 4e can electronically transfer the information by means of an electric cable 4f or other communication device to a visual interface 4g, such as a display.

The processing unit 4 can further comprise additional signal system, such as an acoustic alarm, for alerting the physician or the user in case where the processing unit 4 detects that one parameter is out of a given range of threshold values.

By performing a tight seal around the patient's nose, the nasal cannula allows providing meaningful parameters to the physician such as respiratory rate, inspiratory and expiratory volumes or guidance to optimize the setting of the High Flow Generator HFG. However, this configuration could suffer a lack of tolerance from the patient due to the permanent contact of the nasal cannula around his/her nose. Another embodiment would be to enhance the comfort of wearing the nasal cannula by limiting the areas of contact with the nose of the patient while keeping the most important parameter available, respiratory rate, along with guidance to optimize the setting of the HFG.

This alternative would allow the medical team chosing the type of cannula best suiting to their needs while optimizing the patient's comfort.

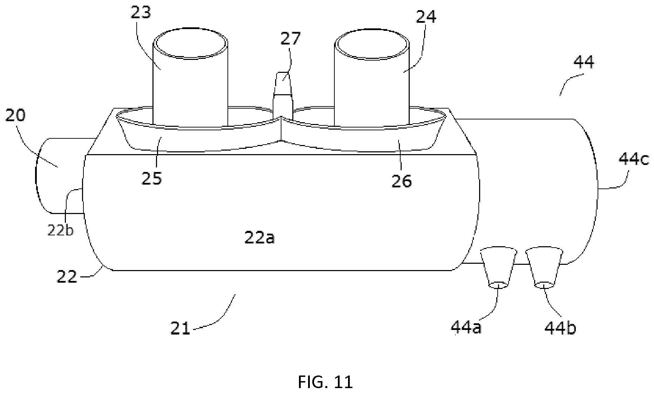

Figure 11 shows another embodiment of the nasal gas-delivery system 21 according to the present invention. It comprises a hollow body 22 having any suitable shape, for example a circular or rectangular section, and providing an inlet 22b receiving a respiratory gas fed by the flexible tubing 20, whereas the outlet orifice 44c, part of a sensing module 44, recovers the expiratory gas of the patient.

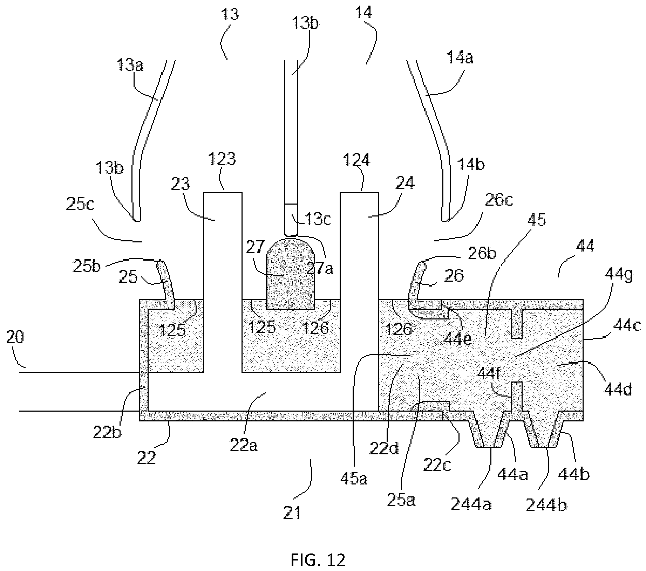

The nasal gas-delivery system 21 further comprises two nasal prongs 23, 24 for delivering the gas to the patient, which comprise two deformable flanges 25, 26 separated by a common wall 27. The flanges 25, 26 are shortened on purpose so as not to come into contact with the patient's nose, as explained below with respect to Fig. 12.

Further, sensing module 44 comprises two ports 44a, 44b, giving access to the flow measurement, as explained in connection with Figure 5.

Fig. 12 is a cross sectional view showing the inner design of the nasal gas-delivery system 21 of Figure 11 and the sensing module 44 fluidly connected to said nasal gas-delivery system 21 and locked and maintained therein by means of the contact between edges 44e, 22c.

The nasal prongs 23, 24 are inserted into the patient's nostrils 13, 14. The inlet 22b of the main hollow body of the nasal gas-delivery system 21, that receives the gas from the flexible tubing 20, is in fluid communication with a first inner chamber 22a that is itself in fluid communication with the inner passages 123, 124 of the prongs 23, 24 so that gas can circulate from the tubing 20 to the prongs 23, 24, via the first inner chamber 22a, and can thereby be delivered to the nostrils 13, 14. Similarly, conduits 125, 126 are in fluid communication with a second inner chamber 22d arranged in the main body of the nasal gas-delivery system 21, that comprises a gas outlet orifice 44c. From the gas outlet orifice 44c, the gases can be easily collected.

Further, the nasal gas-delivery system 21 comprises two flanges 25, 26 which are shortened so as to avoid any contact with the nose of the patient, enhancing the comfort of wearing the cannula. This is made possible in providing the contact point 27a of the flexible wall 27 and the columella 13c higher than the upper edges of the flanges 25, 26, respectively 25b, 26b. A difference of few millimeters, typically 1, 2 or 3 mm will be enough to accommodate various noses variations in a given population (adults, infants...). As a consequence, vents 25c, 26c are created between the lower ends of the alas 13b, 14b and upper edges of the flanges, 25b, 26c respectively.

With such a configuration, the gaseous flow, such as air, conveyed by the tubing 20 enters into the internal chamber 22a of the hollow body 22, travels through the first and second conduits 123, 124 of the pair of prongs 23, 24 and is then delivered to the nostrils 13, 14 of the patient.

During the inhalation phases of the patient, a part of the gaseous flow is inspired by the patient, whereas the rest of the gaseous flow is directed either to ambient through the vents 25c, 26c and to the conduits 125, 126 which are fluidly connected to the vent conduit 22d and subsequently to internal chamber 44d thereby allowing the gas escaping to the atmosphere by venting port(s) 44c. The amount of gas escaping through vents 25c, 26c or venting port 44c will depend on the respective resistance to flow of such ports. For example if the surface area around the nose of vents 25c, 26c is greater than the surface area of the conduits 125, 126, a larger portion of the gas will escape from vents 25b, 26c than venting port 44c, and conversely.

During the exhalation phases of the patient, both gaseous flows coming from the internal chamber 22a of the hollow body 22, on the one hand, and exhaled by the patient, on the other hand, will be vented to the atmosphere either through vents 25c, 26c and the conduits 125,126, vent conduits 22d and subsequently to internal chamber 44d, and venting port or gas outlet orifice 44c, thereby creating a positive expiratory pressure or PEP. The amount of gas vented through ports 25c, 26c, 44c will also depend of the surface ratio explained above.

The nasal gas-delivery 21 can be made of soft silicon material so as to be light for the patient. Further, it preferably comprises one or several straps, a headgear or similar a fixing system (not shown) for maintaining the nasal gas delivery system 21 in position in the nostrils 13, 14 of the patient.

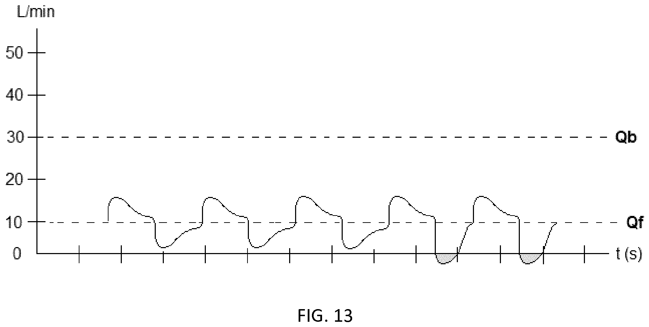

Fig. 13 illustrates the gaseous flow (in L/min) that is measured by unit 4 over the time (in sec). As one can see, the flow is swinging around a mean value Qf (i.e. an average flow value) representing a portion of the flow coming of the HFG 3 by virtue of the spacing 25a, 26a. Upon inhalation, most of the flow coming from the HFG 3 will be inspired by the patient 1 and the instantaneous flow traveling in the chamber 44d will fall below the average flow value. On the opposite, during expiration, the flow of the patient will sum up with the flow of the HFG 3 and the instantaneous flow traveling in the chamber 44d will rise above the average flow value.

As one can see, the last 2 inspirations are greater than the previous ones, indicating for example that the patient has increased his respiratory demand, resulting in a portion where the flow becomes negative. This means that the demand of the patient exceeds the flow delivered by the HFG 3 and that the flow comes backward, that is sucked from ambient air by the patient 1, traveling from inner chamber 44d to exhaust ports 125,126 of the nasal prongs.

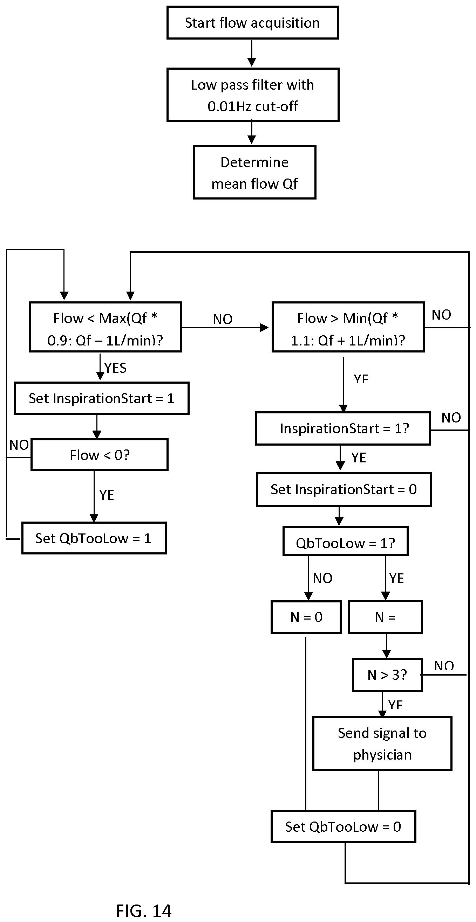

Fig. 14 exhibits an algorithm that could be implemented in unit 4 and operated by the controller 4e to feed the medical team with useful information.

As the flow therapy is started, a low-pass filter with a cut-off frequency within the range of 0.005 to 0.1 Hz, typically 0.01 Hz is applied. This has the benefit of removing the breathing pattern of the patient 1, whose frequency is usually in the range of 0.1 to 1 Hz, and gives access to the average flow Qf that is measured by the unit 4, which, by virtue of the spacing 25a and 26a is inferior to the baseline flow Qb representing the flow delivered by the HFG 3.

Such portion of the algorithm is continuously running in order to take into account any setting or adjustment of the flow that has to be delivered by the High Flow Generator 3.

This value of Qf is then used to define two limits comprising a upper limit and a lower limit, the lower one being slightly lower and the upper one being slightly greater than Qf.

These upper and lower limits will determine whether the patient is inhaling (i.e. lower values) or exhaling (i.e. upper values). The lower value is set as the maximum of 90% of Qf or Qf - 1 L/min, whereas the upper limit is set as the minimum of 110% of Qf or Qf + 1L/min.

For example, if Qf is determined as 5 L/min, the lower limit is 4.5 L/min and the upper limit is 5.5 L/min.

At any moment, once Qf has been determined, the controller 4e verifies the conditions. In case the flow goes below the set lower limit, the condition of an inspiration is detected. It is important to note that in the same period of time, a second algorithm checks whether the flow is negative to determine that the patient had to breath on his/her own due to a demand exceeding the flow Qb delivered by the HFG 3.

When the flow is between the lower and upper limits a specific algorithm will check whether the flow was negative during the previous inspiration, which could expose the patient to respiratory fatigue. In case of 3 occurrences in a row, i.e. 3 consecutives inspiration, a signal will be sent to the medical team, asking them to check the flow delivered by the HFG 3 and increase its value, if necessary.

By counting the number of time an inspiration was detected over a given period, it is easy to determine the respiratory rate of the patient.

Depending on the selection of the nasal cannula, e.g. ensuring tightness or not around of the patient's nose, several information are made available to the physician, such as, at least the respiratory rate of the patient and an inadequacy of his/her needs relating to the set flow of the High Flow Generator 3. Another interesting parameter would be the intra-nasal pressure of the patient. As this parameter results on the flow set by the physician, the geometry of the patient's nose and type of nasal cannula, it is a widely variable parameter, yet essential:

- It represents the PEEP which the patient is exposed to so the physician may want to adjust the flow so as to reach a pressure enhancing the gas exchanges
- It can be harmful if its value is too high, promoting lung distension

The way to make available this useful parameter is described hereafter:

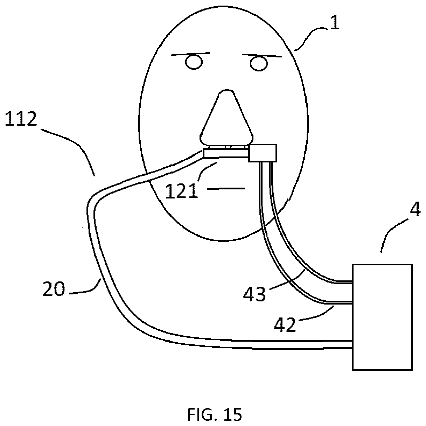

Fig. 15 represents an optimization of Fig. 3, where the High Flow Generator 3 is embedded into the processing unit 4. Processing unit 4 therefore encompasses both the ability to deliver a flow to the patient via the tubing 20 as described earlier and to display information to the user such as respiratory rate, inspiratory and expiratory volumes etc...

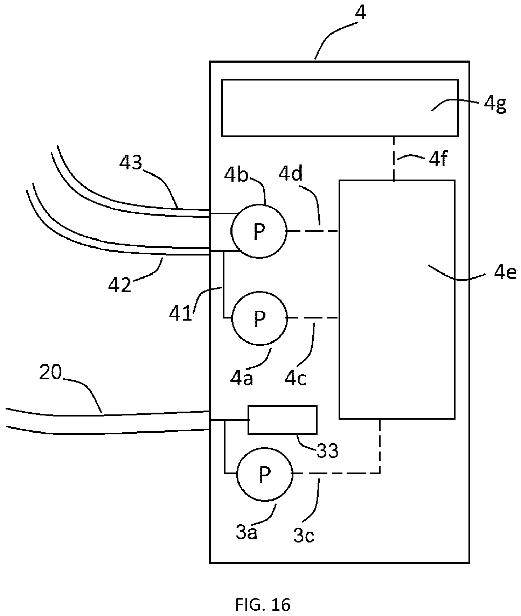

Fig.16 provides the same details as in Fig.7 but also includes the flow generator 33 (not described here), which delivers the flow to the patient. An additional pressure sensor 3a measures the pressure at the inlet of the tubing 21. Such measure is made possible by an electrical connection by the cable 3c to the controller 4e.

As a matter of fact, the tubing 20 and prongs 23, 24 as described in Fig. 6 for instance fluidly connect the pressure sensor 3a to the nasal cavity 13, 14 of the patient. It is understood that these conduits also generate a pressure drop upon transfer of a flow from the generator 33. As the nasal cannula is known, it is possible to get the relationship between the flow that is set by the physician and respective pressure drop operated by the conduits 20, 23, 24. The controller 4e can take into account this pressure drop so as to give access to an estimate of the pressure residing in the nasal cavities 13, 14, e.g. the intra-nasal pressure. This is made by subtracting the pressure measured by the pressure sensor 3a (sending the information to the processing unit 4e by the mean of the cable 3c) and a predefined look-up table, stored within the controller 4e, and associating the pressure drop generated by the conduits 20, 23, 24 to the set flow:

$$Pdrop = f(Flow).$$

This reconstitution of the intra-nasal pressure can then be transmitted to the visual interface 4g so as to be available to the physician. Here also the processing unit 4 can further comprise additional signal system, such as an acoustic alarm, for alerting the physician or the user in case where the processing unit 4 detects that the intra-nasal pressure is out of a given range of threshold values

**Claims**

1. Nasal high flow therapy assembly comprising:

A) a nasal gas-delivery system (21) to be connected to a nose of a patient for delivering a respiratory gas to the patient, comprising:

- a hollow body (22) with an inner chamber (22a) comprising an inlet (22b) for receiving a respiratory gas, and
- a pair of nasal prongs (23, 24) in fluid communication with the inner chamber (22a) of the hollow body (22), each nasal prong (23, 24) comprising a first inner passage (123;

124) fluidly connecting the internal chamber (22a) of the hollow body (22) with a nostril (13, 14) of the patient, and
- the hollow body (22) comprises a pair of second passages (125; 126) for fluidly connecting a nostril (13, 14) with a vent conduit (22d) arranged in the hollow body (22), said vent conduit (22d) comprising one venting port (25a),

charecterized in that the nasal high flow therapy assembly further comprises:
B) a sensing module (44) comprising a hollow body (45) comprising an internal chamber (44d) with an inlet orifice (45a), an outlet orifice (44c), a first port and (44a), a second port (44b) and an inner wall (44f) arranged between the first and second ports (44a, 44b) and radially projecting into the internal chamber (44d) thereby forming a restriction of passage for the gas in said internal chamber (44d), said first and second ports (44a, 44b) each comprising an exit orifice (244a, 244b), the inlet orifice (45a) of the sensing module (44) being fluidly connected to the venting port (25a) of the vent conduit (22d) of the nasal gas-delivery system (21).

2. The assembly according to Claim 1, wherein a pair of flexible flanges (25, 26) are arranged around the nasal prongs (23, 24) of the nasal gas-delivery system (21).

3. The assembly according to Claim 1, wherein the inner wall (44f) radially projecting into the internal chamber (44d) of the nasal gas-delivery system (21) has a disk-shape with a central orifice (44g).

4. The assembly according to Claim 1, wherein the pair of flexible flanges (25, 26) of the nasal gas-delivery system (21) comprises a common flexible wall (27).

5. The assembly according to Claim 1, wherein the nasal gas-delivery system (21) further comprises a fixing system for maintaining the nasal prongs (23, 24) in position into the patient's nostrils (13, 14).

6. The assembly according to Claim 1, wherein the assembly further comprises a processing unit (4) comprising:

- a first pressure sensor (4a) and a second pressure sensor (4b), electrically connected to a controller (4e),
- a controller (4e) for processing information provided by the first and second pressure sensors (4a, 4b),
- the first pressure sensor (4a) and a second pressure sensor (4b) being connected to the internal gas circuit (41), said internal gas circuit (41) being in fluid communication with the first port (44a) of the sensing module (44).

7. The assembly according to Claim 6, wherein the internal gas circuit (41) of the processing unit (4) is in fluid communication with the first port (44a) of the sensing module (44) by a first tubing (42).

8. The assembly according to Claim 6, wherein the second pressure sensor (4b) of the processing unit (4) is a differential pressure sensor.

9. The assembly according to Claim 6, wherein the second pressure sensor (4b) of the processing unit (4) is connected to the first and second ports (44a, 44b) of the sensing module (44) by means of the first (42) tubing and a second (43) tubing.

10. The assembly according to Claim 6, wherein the controller (4e) is electrically connected to a visual interface (4g) for displaying information.

11. The assembly according to Claim 1, wherein the assembly further comprises a high flow generator (3) for delivering a high flow of gas, said high flow generator (3) being in fluid communication with the inlet (22b) of the inner chamber (22a) of the hollow body (22) of the nasal gas-delivery system (21).

12. The assembly according to Claim 11, wherein the high flow generator (3) is fluidly connected to the inlet (22b) of the inner chamber (22a) of the hollow body (22) of the nasal gas-delivery system (21) by a flexible tubing (20).

13. A method of analyzing breathing activity of a patient whose nose is connected to a nasal gas-delivery system (21) of Claim 1, the method comprising the steps of:

a) measuring a baseline respiratory flow $Qb$ flowing through the sensing module (44),
b) defining an upper value of $1.1*Qb$,
c) defining a lower limit of $0.9*Qb$,
d) during patient breathing through the nasal gas-delivery system (21), measuring a breathing associated flow through the sensing module (44),
e) determining if the breathing associated flow through the sensing module (44) is

• below the lower limit, and/or
• above the upper limit,

f) when the breathing associated flow through the sensing module (44) is below the lower limit, determining the net volume of flow attributable to gas drawn off by the patient (Vi0), wherein a preset threshold for Vi0 is defined as indicative of an excessive patient breathing effort,

g) when the breathing associated flow through the sensing module (44) is above the upper limit, determining if the net volume of exhaled flow (Ve) attributable to excess flow from the nasal gas-delivery system (21) exceeds a predetermined amount or a predetermined amount for a predetermined number of times within a predefined period.

14. The method of claim 13, wherein when the breathing associated flow through the sensing module (44) is below the lower limit under step f), further determining the net volume of flow attributable to gas drawn off by the patient (Vi) and

• when the breathing associated flow through the sensing module (44) is negative, determining the net volume of flow coming from ambient room drawn off by the patient (Vi0) and then,
• determining an excessive patient breathing effort when Vi0 is greater than Vi/10.

**Patentansprüche**

1. Nasale Hochdurchfluss-Therapieanordnung, umfassend:

A) ein nasales Gasabgabesystem (21), das mit einer Nase eines Patienten zur Abgabe eines Atemgases an den Patienten verbunden werden soll, umfassend:

- einen Hohlkörper (22) mit einer Innenkammer (22a), die einen Einlass (22b) zur Aufnahme eines Atemgases umfasst, und
- ein Paar von Nasenkanülen (23, 24) in Fluidkommunikation mit der Innenkammer (22a) des Hohlkörpers (22), wobei jede Nasenkanüle (23, 24) einen ersten internen Durchlass (123; 124) umfasst, der die interne Kammer (22a) des Hohlkörpers (22) mit einem Nasenloch (13, 14) des Patienten fluidisch verbindet, und
- der Hohlkörper (22) ein Paar zweiter Durchlässe (125; 126) zur fluidischen Verbindung eines Nasenlochs (13, 14) mit einem Entlüftungskanal (22d) umfasst, der in dem Hohlkörper (22) angeordnet ist, wobei der Entlüftungskanal (22d) eine Entlüftungsanschlussöffnung (25a) umfasst,

**dadurch gekennzeichnet, dass** die nasale Hochdurchfluss-Therapieanordnung weiter umfasst:

B) ein Erfassungsmodul (44), umfassend einen Hohlkörper (45), umfassend eine interne Kammer (44d) mit einer Einlassöffnung (45a), einer Auslassöffnung (44c), einer ersten Anschlussöffnung (44a) und einer zweiten Anschlussöffnung (44b) und einer Innenwand (44f), die zwischen der ersten und zweiten Anschlussöffnung (44a, 44b) angeordnet ist und radial in die interne Kammer (44d) vorsteht, wodurch eine Einschränkung des Durchlasses für das Gas in der internen Kammer (44d) gebildet wird, wobei die erste und zweite Anschlussöffnung (44a, 44b) jeweils eine Austrittsöffnung (244a, 244b) umfassen, wobei die Einlassöffnung (45a) des Erfassungsmoduls (44) mit der Entlüftungsanschlussöffnung (25a) des Entlüftungskanals (22d) des nasalen Gasabgabesystems (21) fluidisch verbunden ist.

2. Anordnung nach Anspruch 1, wobei ein Paar flexibler Flansche (25, 26) rund um die Nasenkanülen (23, 24) des nasalen Gasabgabesystems (21) angeordnet ist.

3. Anordnung nach Anspruch 1, wobei die Innenwand (44f), die radial in die interne Kammer (44d) des nasalen Gasabgabesystems (21) vorsteht, eine Scheibenform mit einer mittleren Öffnung (44g) aufweist.

4. Anordnung nach Anspruch 1, wobei das Paar flexibler Flansche (25, 26) des nasalen Gasabgabesystems (21) eine gemeinsame flexible Wand (27) umfasst.

5. Anordnung nach Anspruch 1, wobei das nasale Gasabgabesystem (21) weiter ein Fixiersystem umfasst, um die Nasenkanülen (23, 24) in den Nasenlöchern (13, 14) des Patienten in Position zu halten.

6. Anordnung nach Anspruch 1, wobei die Anordnung weiter eine Verarbeitungseinheit (4) umfasst, umfassend:

- einen ersten Drucksensor (4a) und einen zweiten Drucksensor (4b), die mit einem Regler (4e) elektrisch verbunden sind,
- einen Regler (4e) zum Verarbeiten von Informationen, die von dem ersten und zweiten Drucksensor (4a, 4b) bereitgestellt werden,
- wobei der erste Drucksensor (4a) und ein zweiter Drucksensor (4b) mit dem internen Gaskreislauf (41) verbunden sind, wobei sich der interne Gaskreislauf (41) in Fluidkommunikation mit der ersten Anschlussöffnung (44a) des Erfassungsmoduls (44) befindet.

7. Anordnung nach Anspruch 6, wobei sich der interne Gaskreislauf (41) der Verarbeitungseinheit (4) durch einen ersten Schlauch (42) in Fluidkommunikation mit der ersten Anschlussöffnung (44a) des Erfassungsmoduls (44) befindet.

8. Anordnung nach Anspruch 6, wobei der zweite Drucksensor (4b) der Verarbeitungseinheit (4) ein Differenzdrucksensor ist.

9. Anordnung nach Anspruch 6, wobei der zweite Drucksensor (4b) der Verarbeitungseinheit (4) mittels des ersten (42) Schlauchs und eines zweiten (43) Schlauchs mit der ersten und zweiten Anschlussöffnung (44a, 44b) des Erfassungsmoduls (44) verbunden ist.

10. Anordnung nach Anspruch 6, wobei der Regler (4e) mit einer visuellen Schnittstelle (4g) zum Anzeigen von Informationen elektrisch verbunden ist.

11. Anordnung nach Anspruch 1, wobei die Anordnung weiter einen Hochdurchfluss-Generator (3) zur Abgabe eines Hochdurchflusses eines Gases umfasst, wobei sich der Hochdurchfluss-Generator (3) in Fluidkommunikation mit dem Einlass (22b) der Innenkammer (22a) des Hohlkörpers (22) des nasalen Gasabgabesystems (21) befindet.

12. Anordnung nach Anspruch 11, wobei der Hochdurchfluss-Generator (3) durch einen flexiblen Schlauch (20) mit dem Einlass (22b) der Innenkammer (22a) des Hohlkörpers (22) des nasalen Gasabgabesystems (21) fluidisch verbunden ist.

13. Verfahren zum Analysieren der Atmungsaktivität eines Patienten, dessen Nase mit einem nasalen Gasabgabesystem (21) nach Anspruch 1 verbunden ist, wobei das Verfahren die folgenden Schritte umfasst:

   a) Messen einer Ausgangsbasis eines Atemdurchflusses Qb, der durch das Erfassungsmodul (44) fließt,
   b) Definieren eines oberen Wertes von 1,1*Qb,
   c) Definieren einer Untergrenze von 0,9*Qb,
   d) während der Patient durch das nasale Gasabgabesystem (21) atmet, Messen eines atmungsassoziierten Durchflusses durch das Erfassungsmodul (44),
   e) Bestimmen, ob der atmungsassoziierte Durchfluss durch das Erfassungsmodul (44)

      • unterhalb der Untergrenze liegt und/oder
      • oberhalb der Obergrenze liegt,

   f) wenn der atmungsassoziierte Durchfluss durch das Erfassungsmodul (44) unterhalb der Untergrenze liegt, Bestimmen des Nettovolumens des Durchflusses, das Gas zugeordnet werden kann, das von dem Patienten (Vi0) entnommen wird, wobei ein voreingestellter Grenzwert für Vi0 als bezeichnend für eine übermäßige Atemanstrengung des Patienten definiert wird,
   g) wenn der atmungsassoziierte Durchfluss durch das Erfassungsmodul (44) oberhalb der Obergrenze liegt, Bestimmen, ob das Nettovolumen des ausgeatmeten Durchflusses (Ve), das einem übermäßigen Durchfluss aus dem nasalen Gasabgabesystem (21) zuordenbar ist, eine vorbestimmte Menge oder eine vorbestimmte Menge für eine vorbestimmte Anzahl von Malen innerhalb einer vordefinierten Dauer übersteigt.

14. Verfahren nach Anspruch 13, wobei, wenn der atmungsassoziierte Durchfluss durch das Erfassungsmodul (44) unterhalb der Untergrenze unter Schritt f) liegt, weiter Bestimmen des Nettovolumens des Durchflusses, das Gas zuordenbar ist, das von dem Patienten (Vi) entnommen wird, und

   • wenn der atmungsassoziierte Durchfluss durch das Erfassungsmodul (44) negativ ist, Bestimmen des Nettovolumens des Durchflusses, das von dem Patienten (Vi0) entnommen wird, das aus dem Umgebungsraum kommt, und dann
   • Bestimmen einer übermäßigen Atemanstrengung des Patienten, wenn Vi0 größer als Vi/10 ist.

**Revendications**

1. Ensemble de thérapie nasale à flux élevé comprenant :

   A) un système nasal de délivrance de gaz (21) à relier au nez d'un patient pour délivrer un gaz respiratoire au patient, comprenant :

      - un corps creux (22) avec une chambre intérieure (22a) comprenant une entrée (22b) pour recevoir un gaz respiratoire, et
      - un couple de canules nasales (23, 24) en communication à fluide avec la chambre intérieure (22a) du corps creux (22), chaque canule nasale (23, 24) comprenant d'un premier passage intérieur (123 ; 124) reliant à fluide la chambre interne (22a) du corps creux (22) à une narine (13, 14) du patient, et
      - le corps creux (22) comprend un couple de seconds passages (125 ; 126) pour relier à fluide une narine (13, 14) à un conduit d'évent (22d) agencé dans le corps creux (22), ledit conduit d'évent (22d) comprenant une seule ouverture d'évacuation (25a),

**caractérisé en ce que** l'ensemble de thérapie nasale à flux élevé comprend en outre :
B) un module de détection (44) comprenant un corps creux (45) comprenant une chambre interne (44d) avec un orifice d'entrée (45a), un orifice de sortie (44c), une première ouverture (44a) et une seconde ouverture (44b) et une paroi intérieure (44f) agencée entre les première et seconde ouvertures (44a, 44b) et en saillie de façon radiale jusque dans la chambre interne (44d) formant de ce fait une restriction de passage pour le gaz dans ladite chambre interne (44d), lesdites première et seconde ouvertures (44a, 44b) comprenant chacune un orifice de sortie (244a, 244b), l'orifice d'entrée (45a) du module de détection (44) étant relié à fluide à l'ouverture d'évacuation (25a) du conduit d'évent (22d) du système nasal de délivrance de gaz (21).

2. Ensemble selon la revendication 1, dans lequel un couple de brides souples (25, 26) est agencé autour des canules nasales (23, 24) du système nasal de délivrance de gaz (21).

3. Ensemble selon la revendication 1, dans lequel la paroi intérieure (44f) en saillie de façon radiale jusque dans la chambre interne (44d) du système nasal de délivrance de gaz (21) a une forme de disque avec un orifice central (44g).

4. Ensemble selon la revendication 1, dans lequel le couple de brides souples (25, 26) du système nasal de délivrance de gaz (21) comprend une paroi souple commune (27).

5. Ensemble selon la revendication 1, dans lequel le système nasal de délivrance de gaz (21) comprend en outre un système de fixation pour maintenir les canules nasales (23, 24) en position dans les narines du patient (13, 14).

6. Ensemble selon la revendication 1, dans lequel l'ensemble comprend en outre une unité de traitement (4) comprenant :

    - un premier capteur de pression (4a) et un second capteur de pression (4b), électriquement reliés à une unité de commande (4e),
    - une unité de commande (4e) pour traiter des informations fournies par les premier et second capteurs de pression (4a, 4b),
    - le premier capteur de pression (4a) et un second capteur de pression (4b) étant reliés au circuit interne de gaz (41), ledit circuit interne de gaz (41) étant en communication à fluide avec la première ouverture (44a) du module de détection (44).

7. Ensemble selon la revendication 6, dans lequel le circuit interne de gaz (41) de l'unité de traitement (4) est en communication à fluide avec la première ouverture (44a) du module de détection (44) par une première tubulure (42).

8. Ensemble selon la revendication 6, dans lequel le second capteur de pression (4b) de l'unité de traitement (4) est un capteur de pression différentielle.

9. Ensemble selon la revendication 6, dans lequel le second capteur de pression (4b) de l'unité de traitement (4) est relié aux première et seconde ouvertures (44a, 44b) du module de détection (44) au moyen de la première (42) tubulure et d'une seconde (43) tubulure.

10. Ensemble selon la revendication 6, dans lequel l'unité de commande (4e) est électriquement reliée à une interface visuelle (4g) pour afficher des informations.

11. Ensemble selon la revendication 1, dans lequel l'ensemble comprend en outre un générateur de flux élevé (3) pour délivrer un flux élevé de gaz, ledit générateur de flux élevé (3) étant en communication à fluide avec l'entrée (22b) de la chambre intérieure (22a) du corps creux (22) du système nasal de délivrance de gaz (21).

12. Ensemble selon la revendication 11, dans lequel le générateur de flux élevé (3) est relié à fluide à l'entrée (22b) de la chambre intérieure (22a) du corps creux (22) du système nasal de délivrance de gaz (21) par une tubulure souple (20).

13. Procédé d'analyse de l'activité respiratoire d'un patient dont le nez est relié à un système nasal de délivrance de gaz (21) selon la revendication 1, le procédé comprenant les étapes de :

    a) mesure d'un flux respiratoire de référence Qb s'écoulant à travers le module de détection (44),
    b) définition d'une valeur supérieure de 1,1*Qb,
    c) définition d'une limite inférieure de 0,9*Qb,
    d) pendant la respiration du patient par l'intermédiaire du système nasal de délivrance de gaz (21), mesure d'un flux associé à la respiration à travers le module de détection (44),
    e) détermination si le flux associé à la respiration à travers le module de détection (44) est

    • au-dessous de la limite inférieure, et/ou
    • au-dessus de la limite supérieure,

    f) quand le flux associé à la respiration à travers le module de détection (44) est au-dessous de la limite inférieure, détermination du volume net de flux attribuable au gaz aspiré par le patient (ViO), dans lequel un seuil défini à l'avance pour Vi0 est défini comme indicatif d'un effort de respiration excessif du patient,

g) quand le flux associé à la respiration à travers le module de détection (44) est au-dessus de la limite supérieure, détermination si le volume net de flux exhalé (Ve) attribuable à un flux excessif en provenance du système nasal de délivrance de gaz (21) dépasse une quantité prédéterminée ou une quantité prédéterminée pendant un nombre prédéterminé de fois dans les limites d'une période prédéfinie.

14. Procédé selon la revendication 13, dans lequel lorsque le flux associé à la respiration à travers le module de détection (44) est au-dessous de la limite inférieure sous l'étape f), détermination supplémentaire du volume net de flux attribuable au gaz aspiré par le patient (Vi), et

• quand le flux associé à la respiration à travers le module de détection (44) est négatif, détermination du volume net de flux provenant de la pièce ambiante aspiré par le patient (ViO), et ensuite
• détermination d'un effort de respiration excessif du patient quand Vi0 est plus grand que Vi/10.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

```
┌─────────────────────────┐
│  Start flow acquisition │
└─────────────────────────┘
             │
             ▼
┌─────────────────────────┐
│    Low pass filter with │
│      0.01Hz cut-off     │
└─────────────────────────┘
             │
             ▼
┌─────────────────────────┐
│        Determine        │
│      mean flow Qf       │
└─────────────────────────┘
```

```
┌──────────────────┐   NO    ┌──────────────────┐   NO
│ Flow < Max(Qf *  │────────▶│ Flow > Min(Qf *  │────────
│ 0.9: Qf – 1L/min)?│        │ 1.1: Qf + 1L/min)?│
└──────────────────┘         └──────────────────┘
         │ YES                        │ YF
         ▼                            ▼
┌──────────────────────┐    ┌──────────────────────┐   NO
│ Set InspirationStart=1│    │  InspirationStart = 1? │────────
└──────────────────────┘    └──────────────────────┘
         │                            │ YE
    NO   ▼                            ▼
┌──────────────────┐        ┌──────────────────────┐
│    Flow < 0?     │        │  Set InspirationStart = 0 │
└──────────────────┘        └──────────────────────┘
         │ YE                         │
         ▼                            ▼
┌──────────────────┐        ┌──────────────────┐
│  Set QbTooLow = 1│        │   QbTooLow = 1?  │
└──────────────────┘        └──────────────────┘
                              NO │        │ YE
                                 ▼        ▼
                           ┌────────┐  ┌────────┐
                           │ N = 0  │  │  N =   │
                           └────────┘  └────────┘
                                          │
                                          ▼
                                   ┌────────────┐   NO
                                   │   N > 3?   │────────
                                   └────────────┘
                                          │ YF
                                          ▼
                                   ┌────────────┐
                                   │ Send signal to │
                                   │   physician    │
                                   └────────────┘
                                          │
                                          ▼
                                   ┌──────────────────┐
                                   │ Set QbTooLow = 0 │
                                   └──────────────────┘
```

FIG. 14

FIG. 15

FIG. 16

**EP 3 246 063 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2075023 A1